# FASCICULE DE BREVET EUROPEEN

(11) **EP 1 632 199 B1**
(45) Date de publication et mention de la délivrance du brevet: **29.06.2011**
(21) Numéro de dépôt: 05356146.0
(22) Date de dépôt: 05.09.2005
(51) Int. Cl.: A61F 2/28, A61B 17/064

(54) **Implant de fixation d'un greffon osseux au sein d'une articulation en vue d'assurer l'arthrodèse de l'articulation**
Implantat zur Fixierung eines Knochentransplantats innerhalb eines Gelenks um die Arthrodese des Gelenks sicherzustellen
Implant for fixing of an osseous graft within a joint to assure the arthrodesis of the joint

(30) Priorité: 06.09.2004 FR 0409427
(43) Date de publication de la demande: 08.03.2006
(73) Titulaire: Newdeal, 69006 Lyon (FR)
(72) Inventeur: Kofoed, Hakon, 2920 Charlottenlund (DK)
(74) Mandataire: Martin, Didier Roland Valéry

(56) Documents cités:
- EP-A- 0 599 766
- WO-A-03/007839
- US-A- 4 723 540
- US-A- 5 425 490

## Description

La présente invention se rapporte au domaine technique général des implants de fixation chirurgicaux, et en particulier au secteur des implants de fixation destinés à être utilisés pour réaliser une arthrodèse.

La présente invention concerne plus particulièrement un implant de fixation d'un greffon osseux disposé entre les os situés de part et d'autre d'une fente articulaire, en vue d'assurer l'arthrodèse d'une articulation.

L'implant de fixation conforme à l'invention est spécifiquement conçu pour réaliser une arthrodèse d'une articulation, notamment, mais non exclusivement, l'articulation de la cheville.

Une arthrodèse est une intervention chirurgicale destinée à supprimer presque complètement la mobilité d'une articulation en provoquant une « *fusion osseuse* »*.* Une telle intervention chirurgicale peut s'avérer nécessaire dans le cas où le patient souffre d'arthrose sévère et finale, ou encore lorsque les cartilages des os formant l'articulation sont très abîmés.

Pour réaliser de telles interventions, il est connu de réséquer les surfaces cartilagineuses abîmées de l'articulation de manière à mettre en contact, par compression, les surfaces osseuses en regard des os formant l'articulation, permettant ainsi l'ostéosynthèse.

Toutefois, une telle méthode peut conduire à un raccourcissement du membre concerné, ce qui n'est bien évidemment pas souhaitable, non seulement du point de vue esthétique, mais également en raison des complications (claudication excessive par exemple) que cela peut entraîner.

Pour pallier ces inconvénients, il est connu de ménager un logement de part et d'autre de la fente articulaire en découpant des fragments d'os au niveau des extrémités en vis-à-vis des os formant l'articulation. On vient ensuite combler ce logement, de préférence cylindrique, avec un greffon osseux, tel qu'une carotte osseuse sensiblement cylindrique prélevée sur le même patient, par exemple au niveau de la crête iliaque.

Cette manipulation permet ainsi d'éliminer les surfaces cartilagineuses dégradées et de remplacer les extrémités d'os abîmées par un greffon osseux sain sans raccourcir le membre concerné. On vient ensuite fixer le greffon osseux relativement aux os formant l'articulation en vue de permettre l'ostéosynthèse entre le greffon osseux d'une part et les os d'autre part, assurant ainsi l'arthrodèse de l'articulation.

Différents implants de fixation peuvent être utilisés en vue d'assurer la fixation du greffon osseux et l'arthrodèse de l'articulation. Ainsi, dans le cas d'une articulation entre un premier et un deuxième os, on utilise couramment un clou de Steinman. Le clou de Steinman se présente sous la forme d'une broche allongée de longueur suffisante pour traverser successivement le premier os de l'articulation, le greffon osseux et le deuxième os de l'articulation. De tels implants de fixation, s'ils permettent d'obtenir des résultats intéressants en matière d'immobilisation de l'articulation, souffrent néanmoins d'inconvénients non négligeables.

Tout d'abord, ces implants de fixation requièrent, pour leur mise en place, une incision supplémentaire s'ajoutant à l'incision déjà réalisée en vue de ménager le logement destiné à recevoir le greffon osseux. Cette incision supplémentaire a pour effet d'augmenter significativement le risque d'infections et de complications opératoires ou post-opératoires.

En outre, les implants de fixation du type clou de Steinman doivent généralement être disposés de manière à s'étendre en oblique ou perpendiculairement par rapport à la fente articulaire en vue d'assurer un maintien efficace de l'articulation. Pour certaines articulations, telles que l'articulation de la cheville, une telle orientation de l'implant n'est pas souhaitable car il existe un risque que lors de la mise en charge du patient, et notamment lors de la marche, l'implant traverse le cortex plantaire du calcanéum et fasse saillie à l'extérieur du faciès plantaire.

Par ailleurs, si l'implant de fixation est mal positionné, il peut également entraîner un endommagement des tissus mous du faciès plantaire. Or, les implants de fixation tels que les clous de Steinman peuvent s'avérer difficiles à positionner, notamment lorsque les articulations mettent en jeu des os de petites dimensions. Ainsi, en cas de mauvaise orientation de l'implant, il peut arriver que le greffon osseux ne soit pas maintenu, l'implant ainsi monté étant alors quasiment inopérant.

Le document US-5,425,490 (GOBLE) décrit par ailleurs un implant chirurgical comprenant une agrafe chirurgicale présentant deux jambes d'ancrage reliées par un élément de connexion et destinées à être introduites dans un os, ainsi qu'une rondelle conçue pour être placée entre lesdites jambes d'ancrage afin de venir maintenir un ligament contre la surface dudit os.

Les objets assignés à l'invention visent par conséquent à porter remède aux différents inconvénients énumérés précédemment et à proposer un nouvel implant de fixation d'un greffon osseux disposé entre les os situés de part et d'autre d'une fente articulaire, en vue d'assurer l'arthrodèse d'une articulation, qui permette d'assurer de façon simple un maintien particulièrement efficace et stable de l'articulation sans risquer d'endommager d'une part le cortex, et d'autre part les tissus mous voisins des os formant l'articulation.

Un autre objet de l'invention vise à proposer un nouvel implant de fixation convient particulièrement pour immobiliser un greffon fragmenté et/ou fissuré.

Un autre objet de l'invention vise à proposer un nouvel implant de fixation adapté pour résister efficacement aux sollicitations mécaniques exercées sur l'articulation, notamment lors de la marche dans le cas de l'articulation de la cheville.

Un autre objet de l'invention vise à proposer un nouvel implant de fixation ne nécessitant pas d'incision supplémentaire pour sa mise en place.

Un autre objet de l'invention vise à proposer un nouvel implant de fixation qui soit moins intrusif que les implants connus.

Un autre objet de l'invention vise à proposer un nouvel implant de fixation présentant une structure et une forme adaptées à l'anatomie de l'articulation.

Un autre objet de l'invention vise à proposer un nouvel implant de fixation permettant un maintien solide et confortable de l'articulation.

Un autre objet de l'invention vise à proposer un nouvel implant de fixation dont la manipulation est facilitée et qui permet de réduire les erreurs opératoires.

Les objets assignés à l'invention sont atteints à l'aide d'un implant de fixation d'un greffon osseux disposé entre les os situés de part et d'autre d'une fente articulaire, en vue d'assurer l'arthrodèse d'une articulation, ledit implant de fixation étant caractérisé en ce qu'il comporte :
- au moins deux éléments d'ancrage destinés à être introduits dans les os, et pourvus d'une extrémité proximale et d'une extrémité distale, ladite extrémité distale étant adaptée pour être introduite dans les os, lesdits éléments d'ancrage étant reliés entre eux par au moins un élément de connexion s'étendant à l'extérieur de l'articulation,
- un moyen d'immobilisation du greffon osseux, disposé entre les éléments d'ancrage et relié à l'élément de connexion de manière à assurer, en coopération avec les éléments d'ancrage, le blocage du greffon osseux vis-à-vis des os de l'articulation et vice-versa,
le moyen d'immobilisation étant formé par une plaque, qui est formée comme définie dans la revendication 1.

D'autres particularités et avantages de l'invention apparaîtront plus en détails à la lecture de la description qui suit, et à l'aide des dessins annexés fournis à titre purement illustratif et non limitatif, dans lesquels :
- La figure 1 illustre, selon une vue en perspective, une articulation de la cheville avec un greffon osseux disposé entre les os situés de part et d'autre d'une fente articulaire.
- La figure 2 illustre, selon une vue en perspective, un implant de fixation conforme à l'invention dans sa position fonctionnelle de maintien de l'articulation.
- La figure 3 illustre, selon une vue en perspective, un premier mode de réalisation d'un implant de fixation conforme à l'invention.
- La figure 4 illustre, selon une vue de côté, un autre mode de réalisation d'un implant de fixation non représentatif de l'invention.
- La figure 5 illustre, selon une vue de face, un implant de fixation conforme à l'invention dans sa position fonctionnelle de compression du greffon osseux et de maintien de l'articulation.
- La figure 6 illustre, selon une vue en coupe suivant la ligne A-A illustrée sur la figure 5, l'implant de fixation illustré sur la figure 5.
- La figure 7 illustre, selon une vue en perspective, une articulation de cheville et un greffon osseux disposé entre les os situés de part et d'autre de la fente articulaire, le greffon osseux étant formé par deux fragments séparés par la fente articulaire.
- La figure 8 illustre, selon une vue en perspective, un implant de fixation conforme à l'invention mis en place au sein de l'articulation illustrée sur la figure 7.
- La figure 9 illustre, selon une vue de côté en perspective, un mode de réalisation de l'implant de fixation conforme à l'invention, pourvu d'un organe de compression.
- La figure 10 illustre, selon une vue de côté en perspective, une variante de réalisation améliorée de l'implant de fixation pourvu d'un organe de compression conforme à l'invention.
- Les figures 11 à 13 illustrent, selon des vues de côté en perspective, différents modes de réalisation d'un organe de compression conforme à l'invention.
- La figure 14 illustre, selon une vue schématique, un implant conforme à un autre mode de réalisation de l'invention.

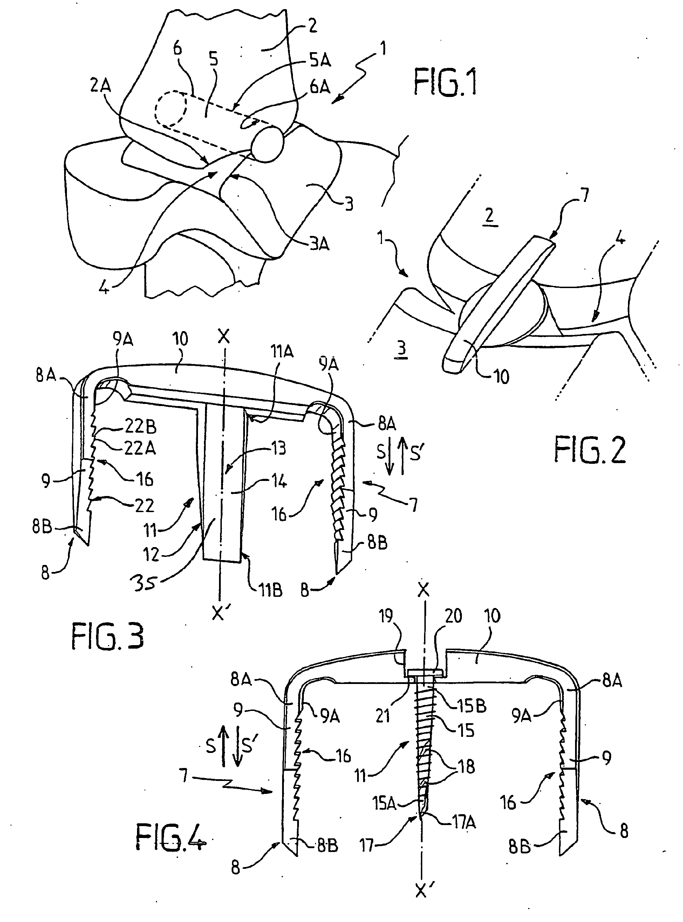

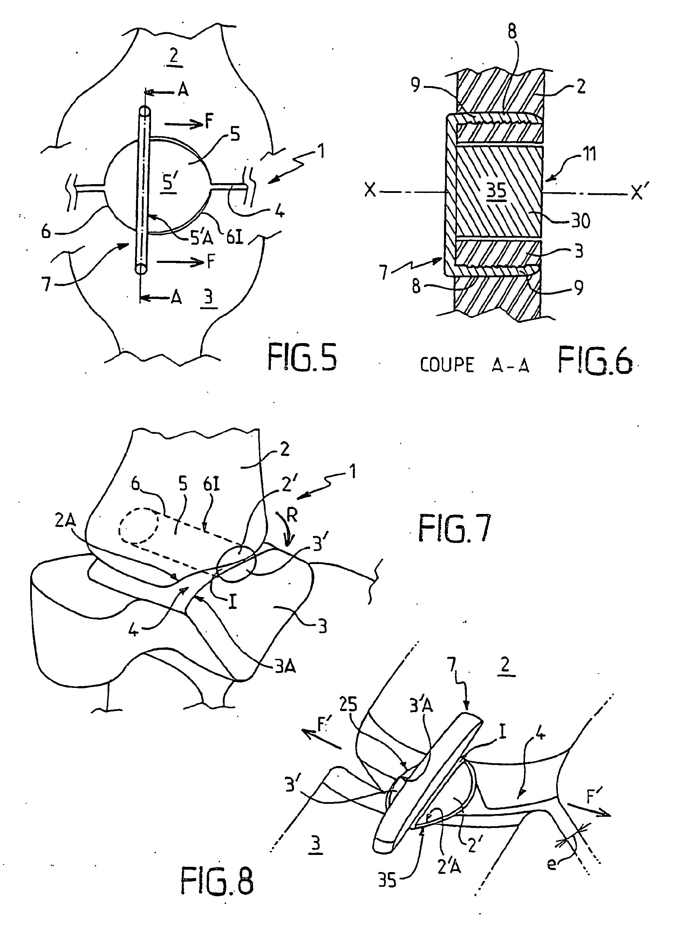

Les figures 1 et 7 illustrent deux méthodes chirurgicales permettant de réaliser une arthrodèse de l'articulation de la cheville.

L'arthrodèse d'une articulation devient nécessaire lorsque l'articulation se trouve dans un état si dégradé que d'autres interventions chirurgicales moins sévères, telles que par exemple celles consistant à poser des prothèses, s'avèreraient inefficaces. Il devient nécessaire, dans ce cas, d'immobiliser complètement l'articulation. La présente invention est illustrée dans le cas d'une articulation de la cheville mais pourrait s'appliquer à tous types d'articulations du corps humain ou animal.

Une articulation abîmée se caractérise notamment par l'état des cartilages des os délimitant la fente articulaire. En cas d'arthrose sévère, ces cartilages sont particulièrement usés et peuvent entraîner des douleurs ou encore une inflammation de l'articulation.

Les figures 1 et 7, illustrent une articulation 1 formée par au moins deux os, à savoir un premier os 2, et un deuxième os 3 situés de part et d'autre d'une fente articulaire 4. Toutefois, l'articulation 1 pourrait bien évidemment comporter un troisième os, par exemple situé entre le premier os 2 et le deuxième os 3, et ce sans sortir du cadre de l'invention.

Plusieurs techniques d'arthrodèse peuvent être envisagées mais l'implant de fixation conforme à l'invention est plus spécifiquement conçu pour être utilisé en vue de réaliser une arthrodèse à l'aide d'un greffon osseux 5 disposé entre les os 2, 3 situés de part et d'autre de la fente articulaire 4. Une première méthode connue consiste ainsi à ménager un logement 6, par exemple cylindrique, dans les extrémités 2A, 3A des os 2, 3 délimitant la fente articulaire 4.

Selon cette première méthode connue, les fragments d'os contenus dans le logement 6 sont extraits et on vient prélever une carotte osseuse dans une autre partie du corps du patient, par exemple au niveau de la crête iliaque, en vue de l'introduire au sein du logement 6 une fois les fragments d'os retirés. Cette carotte constitue, après sa mise en place au sein du logement 6, un greffon osseux 5, sensiblement plein, c'est-à-dire ni-creux, ni-fendu, qu'il convient de fixer relativement aux os 2, 3 en vue d'assurer l'ostéosynthèse entre le greffon osseux 5 et les os 2,3. Précisément, l'ostéosynthèse est réalisée entre la surface osseuse externe saignante 5A du greffon osseux 5 et la surface de coupe 6A, également osseuse, des os 2, 3. Au sens de l'invention, la surface osseuse externe saignante 5A du greffon osseux 5 correspond à la surface selon laquelle le greffon osseux 5 a été découpé. Dans le cas d'une carotte cylindrique, la surface osseuse externe saignante 5A correspond donc à la surface latérale de la carotte. La surface de coupe 6A correspond sensiblement, au sens de l'invention, à la paroi interne 6I du logement 6.

Une variante de cette méthode a été développée par le demandeur. Comme la méthode précédente, la méthode du demandeur consiste à ménager un logement 6 de préférence cylindrique, de part et d'autre de la fente articulaire 4. En revanche, le logement 6 n'est pas évidé de son contenu, c'est-à-dire que l'on ne retire pas les fragments d'os 2', 3' découpés respectivement dans les extrémités des os 2, 3 situés de part et d'autre de la fente articulaire 4. Les fragments d'os 2', 3', juxtaposés au sein du logement 6 et séparés par un interstice I provenant par exemple de la fente articulaire 4 constituent alors le greffon osseux 5. Les surfaces cartilagineuses usées de l'articulation sont ainsi situées de part et d'autre de l'interstice I séparant les fragments d'os 2', 3' et donc au centre du greffon osseux 5.

La méthode du demandeur consiste ensuite à déplacer le greffon osseux 5, formé par les fragments d'os 2', 3', au sein de son logement 6, en le faisant tourner sur lui-même par exemple d'un quart de tour suivant le sens de rotation R indiqué sur la figure 7. De cette façon, on vient mettre en regard la surface externe osseuse saignante 2'A du premier fragment d'os 2' en regard de la surface de coupe 3S, également osseuse, du deuxième os 3. De la même façon, on vient mettre en regard la surface externe osseuse saignante 3'A du deuxième fragment d'os 3' en regard de la surface de coupe 2S du premier os 2. Ceci permet ainsi l'ostéosynthèse des fragments d'os 2' et 3' avec respectivement les os 3 et 2 et plus généralement l'ostéosynthèse du greffon osseux 5 avec les os 2,3.

Au sens de l'invention, l'expression « *greffon osseux* » fait donc référence soit à un transplant osseux, c'est-à-dire un morceau d'os, de préférence monobloc, prélevé dans une partie du corps située à distance de l'articulation 1 et introduit au sein du logement 6, soit à un assemblage de fragments d'os 2', 3' découpés au sein de l'articulation 1 lors de la réalisation du logement 6, et à qui on a fait subir un déplacement, tel qu'une rotation R au sein du logement 6. Le greffon osseux 5 constitue donc un ensemble unitaire, formé soit par un fragment osseux monobloc, soit par une pluralité de fragments osseux juxtaposés au sein du logement 6.

Les figures 3 à 6 illustrent plusieurs variantes de réalisation d'un implant de fixation 7, destiné notamment à être utilisé pour fixer le greffon osseux 5, monobloc, illustré sur la figure 1.

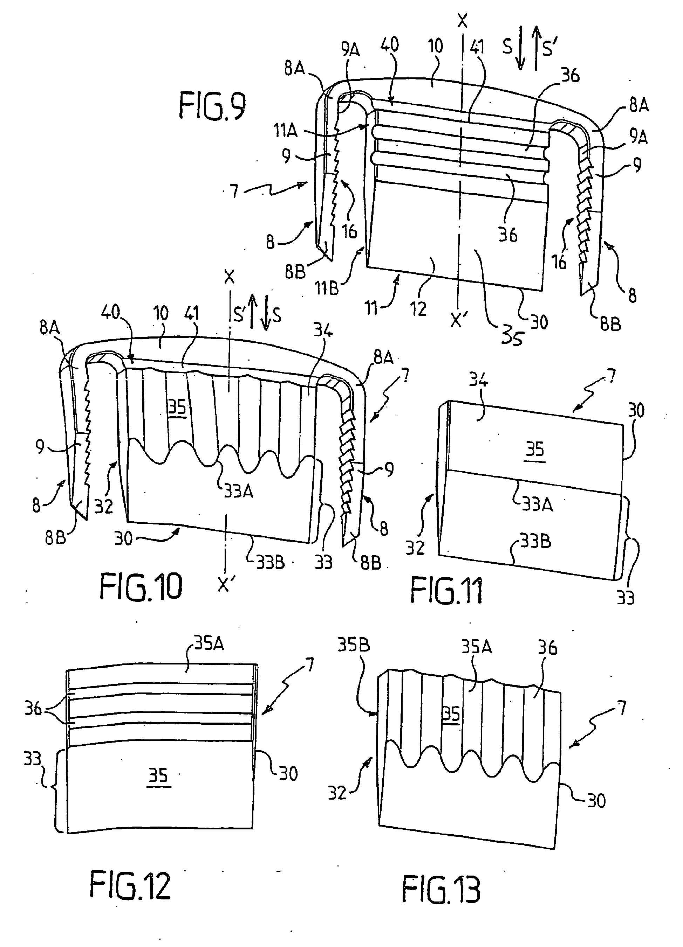

Les figures 9 à 14 illustrent d'autres variantes de réalisation de l'implant de fixation 7 destiné notamment à être utilisé pour assurer la fixation du greffon osseux 5 formé par les fragments d'os 2', 3' illustrés sur la figure 7.

Selon l'invention, l'implant de fixation 7 comporte au moins deux éléments d'ancrage 8 destinés à être introduits dans les os 2, 3. De façon préférentielle, l'implant de fixation 7 comporte autant d'éléments d'ancrage 8 que l'articulation comporte d'os 2, 3. Ainsi, si l'articulation 1 est formée par deux os 2, 3, l'implant de fixation comportera de préférence deux éléments d'ancrage 8, tel que cela est représenté sur les figures 3, 4 et 9. Il est toutefois bien évidemment envisageable de munir l'implant de fixation de plusieurs éléments d'ancrage pour un même os et ce, sans sortir du cadre de l'invention.

Chaque élément d'ancrage 8 s'étend préférentiellement entre une extrémité proximale 8A et une extrémité distale 8B. L'extrémité distale 8B est en outre adaptée pour être introduite dans les os 2, 3 et est à cet effet préférentiellement effilée, ou en forme de pointe. Grâce à cette mesure technique, chaque élément d'ancrage 8 présente un caractère auto-perforant, permettant la pénétration de l'élément d'ancrage 8 dans l'os.

Les éléments d'ancrage 8 sont avantageusement reliés entre eux par l'intermédiaire d'au moins un élément de connexion 10. Tel que cela est illustré sur les figures 2 et 8, l'élément de connexion 10 s'étend à l'extérieur de l'articulation 1 et chevauche la fente articulaire 4, formant ainsi un pont de connexion sensiblement rigide entre les éléments d'ancrage 8 et donc entre les os 2, 3. Ce pont de connexion 10 confère ainsi à l'implant de fixation 7 sa rigidité structurelle, ce qui lui permet de mieux résister aux différentes sollicitations mécaniques auxquelles est soumise l'articulation 1. L'élément de connexion 10 étant disposé, dans sa position fonctionnelle, entre l'articulation 1 et les tissus mous (non-représentés), il présente de préférence des bords arrondis qui lui confèrent un caractère sensiblement atraumatique vis-à-vis des tissus mous environnants.

De façon préférentielle, les éléments d'ancrage 8 sont formés par des branches d'ancrage 9 dont les extrémités distales 8B sont sensiblement biseautées de manière à faciliter leur pénétration dans les tissus osseux.

Avantageusement, les éléments d'ancrage 8, notamment les branches d'ancrage 9, s'étendent sensiblement parallèlement à la fente articulaire 4, et sont sensiblement perpendiculaires à l'élément de connexion 10. L'implant de fixation 7 présente alors avantageusement une forme en U, les branches du U étant formées par les branches d'ancrage 9, et la base du U étant formée par l'élément de connexion 10.

Il est toutefois bien évidemment envisageable de réaliser un implant de fixation dont les branches d'ancrage 9 s'étendent de façon oblique et non perpendiculaire par rapport à l'élément de connexion 10 et de manière à se rapprocher l'une de l'autre. Les branches d'ancrage 9 peuvent ainsi avantageusement être réalisées à partir d'un matériau à mémoire de forme de manière à se resserrer, et à se rapprocher l'une de l'autre une fois introduites dans les os 2, 3, afin d'assurer une compression efficace des os 2, 3 l'un contre l'autre.

Selon l'invention, l'implant de fixation 7 comporte également un moyen d'immobilisation 11 du greffon osseux 5, disposé entre les éléments d'ancrage 8 et relié à l'élément de connexion 10 de manière à assurer, en coopération avec les éléments d'ancrage 8, le blocage du greffon osseux 5 vis-à-vis des os 2, 3 de l'articulation 1 et inversement.

L'expression « en coopération » fait référence au fait que le moyen d'immobilisation 11 agit conjointement avec les éléments d'ancrage 8 pour immobiliser le greffon osseux 5 vis-à-vis d'une part des éléments d'ancrage 8 et d'autre part des os 2, 3 de l'articulation 1. Avantageusement, le moyen d'immobilisation 11 s'étend, longitudinalement, suivant une direction longitudinale X-X' sensiblement parallèle à la fente articulaire 4 et ce, contrairement aux dispositifs de l'art antérieur, tels que les clous de Steinman, qui s'étendent de façon sensiblement perpendiculaire ou en oblique par rapport à la fente articulaire.

Le moyen d'immobilisation 11 présente bien entendu une forme et des dimensions adaptées pour assurer une immobilisation stable et fiable du greffon. En cela, le moyen d'immobilisation n'est pas un simple moyen d'indexation en position de l'implant relativement au greffon, mais bien un moyen d'encastrement mécanique de l'implant au greffon.

Le moyen d'immobilisation 11 est également distinct et exogène du greffon et adapté pour coopérer avec ce dernier. Le moyen d'immobilisation 11 ne forme donc pas directement par lui-même, au sens de l'invention, un substrat de croissance ou de régénération osseuse ou tissulaire, mais bien un organe interagissant mécaniquement avec le greffon pour bloquer ce dernier.

Le moyen d'immobilisation 11 s'étend ainsi avantageusement, suivant la direction longitudinale X-X', entre une partie proximale 11A reliée à l'élément de connexion 10 et une partie distale 11B disposée à l'opposé de la partie proximale 11A. Le moyen d'immobilisation 11 est ainsi disposé de façon perpendiculaire à l'élément de connexion 10.

De façon préférentielle, les branches d'ancrage 9 s'étendent longitudinalement de façon sensiblement parallèle à la direction longitudinale X-X' d'extension du moyen d'immobilisation 11. En outre, les branches d'ancrage 9 présentent avantageusement, sur leur longueur, une épaisseur variable qui décroît entre leur extrémité proximale 8A et leur extrémité distale 8B de manière à faciliter leur pénétration dans les os 2, 3.

Tel que cela est illustré sur la figure 3, les branches d'ancrage 9 ont préférentiellement la même longueur que le moyen d'immobilisation 11. Il est toutefois bien évidemment envisageable de réaliser des branches d'ancrage 9 de longueurs différentes. Ainsi, il est envisageable de réaliser un implant de fixation 7 muni de branches d'ancrage 9 sensiblement plus longues que le moyen d'immobilisation 11 (figure 4).

Avantageusement, le moyen d'immobilisation 11 comporte un organe d'introduction 12 au sein du greffon osseux 5. L'organe d'introduction 12 est ainsi adapté pour pénétrer soit à l'intérieur d'un greffon osseux 5 monobloc, formé par un unique fragment d'os (figure 2), soit dans l'interstice I séparant les fragments d'os 2', 3' au sein du greffon osseux 5 (figures 7 et 8). A cet effet, la partie distale 11B du moyen d'immobilisation 11, qui forme l'organe d'introduction 12, est de préférence effilée ou biseautée.

Afin d'assurer un maintien efficace du greffon osseux 5 et de permettre l'ostéosynthèse avec les os 2, 3, le moyen d'immobilisation 11 comporte avantageusement des moyens de blocage en rotation 13 adaptés pour empêcher la rotation du greffon osseux 5 autour de l'implant de fixation 7, et vice-versa.

Tel que cela est illustré sur la figure 3, les moyens de blocage en rotation 13 sont avantageusement formés par au moins un méplat 14, ménagé le long du moyen d'immobilisation 11. Le moyen d'immobilisation 11 peut ainsi se présenter sous la forme d'une pointe comportant au moins une face externe sensiblement plane formant le méplat 14.

Conformément à l'invention, tel que cela est illustré aux figures 3, 6 et 9 à 14, le moyen d'immobilisation 11 est formé par au moins une plaque 35, c'est à dire par un élément bidimensionnel. Par « *élément bidimensionnel* »*,* on désigne ici un élément de forme aplatie, dont l'épaisseur est faible en regard de sa longueur et de sa largeur. En d'autres termes, un tel moyen d'immobilisation 11 présente une forme de lame et s'étend majoritairement selon deux directions de l'espace, et non selon essentiellement une seule direction de l'espace, comme la vis 15 décrite plus en détails ci-après.

La plaque 35 peut, au sens de l'invention, présenter une forme relativement étalée, comme dans la variante des figures 9 à 14, ou plutôt élancée, comme dans la variante de la figure 3.

La mise en oeuvre d'un moyen d'immobilisation 11 formé par une plaque 35 permet un excellent maintien du greffon osseux 5, et autorise en particulier l'utilisation d'un greffon fissuré, fendu ou même brisé en plusieurs fragments. Dans ce cas, la mise en oeuvre d'un moyen d'immobilisation 11 en forme de plaque permet en effet d'assurer un blocage relatif des fragments suffisamment robuste et stable pour résister aux charges auxquelles peut être soumise l'articulation (en particulier lorsque cette dernière est une articulation de cheville, sur laquelle s'exerce le poids du corps du patient).

La plaque 35 peut être d'épaisseur sensiblement uniformément constante. Dans le cas où le greffon est formé de plusieurs fragments, les dimensions de la plaque, et en particulier son épaisseur, seront de préférence choisies en fonction de l'espace libre entre les fragments de telle sorte que la plaque occupe un volume suffisant pour bloquer les fragments.

La plaque 35 peut cependant être éventuellement constituée de plusieurs tronçons présentant chacun une épaisseur sensiblement constante et différente de l'épaisseur des autres tronçons. Dans ce cas, le raccordement entre chaque tronçon peut être brusque, et se présenter par exemple sous la forme d'un épaulement ou d'une « *marche* »*.*

Il est également envisageable, dans une variante préférentielle de réalisation illustrée aux figures 3 et 9 à 13, et décrite plus en détails ci-après, que la plaque 35 présente une forme «*en coin*», biseautée, c'est à dire dont l'épaisseur augmente progressivement, selon la direction longitudinale, sur au moins une partie de la plaque, de son extrémité distale 11B vers son extrémité proximale 11A.

Dans ce cas, décrit plus en détails dans ce qui suit, le moyen d'immobilisation forme (ou est formé) par un organe de compression 30.

Selon une variante de réalisation non conforme à l'invention illustrée sur la figure 4, le moyen d'immobilisation 11 peut toutefois être formé par une vis 15 de préférence auto-foreuse et auto-taraudante, et pourvue à cet effet de moyens de préparation 17 formés par au moins une dent 17A s'étendant sensiblement axialement suivant la direction longitudinale X-X'.

Avantageusement, la vis 15 comporte également des gorges 18 ménagées sur sa longueur de manière à permettre l'évacuation progressive de la matière osseuse excédentaire lors de son vissage à l'intérieur du greffon osseux 5.

Le moyen d'immobilisation 11 peut être montée de façon amovible sur l'élément de connexion 10. Selon un mode de réalisation non représentatif de l'invention et illustré sur la figure 4, l'élément de connexion 10 peut être pourvu d'un trou débouchant 19, préférentiellement ménagé sensiblement au centre de l'élément de connexion 10 et adapté pour recevoir le moyen d'immobilisation 11. Ainsi, la vis 15 peut avantageusement comporter, vers son extrémité proximale 15B, une tête 20 destinée à venir en appui contre un épaulement 21, formant en butée, ménagé au sein du trou débouchant 19.

Selon une autre variante de réalisation de l'invention illustrée sur les figures 3, 9 et 10, le moyen d'immobilisation 11 est avantageusement solidarisé à demeure avec l'élément de connexion 10 et par exemple venu de matière avec ce dernier, formant ainsi un ensemble monobloc.

De façon préférentielle, et tel que cela est illustré aux figures 3 et 4, les moyens d'ancrage 8 sont formés par deux branches d'ancrage 9 latérales et espacées, disposées en vis-à-vis de part et d'autre de l'élément de connexion 10, parallèlement l'une par rapport à l'autre. Les branches d'ancrage 9 sont de préférence venue de matière avec l'élément de connexion 10 mais peuvent bien évidemment être formées par des pièces distinctes de l'élément de connexion 10, et solidarisées à ce dernier, par exemple à l'aide de vis de fixation (variante non représentée). De façon particulièrement avantageuse les branches d'ancrage 9 sont préférentiellement identiques et disposées symétriquement de part et d'autre du moyen d'immobilisation 11.

En outre, le moyen d'immobilisation 11 est avantageusement formé par une branche centrale sensiblement parallèle aux branches d'ancrage 9 latérales, de manière à s'étendre de façon perpendiculaire par rapport à l'élément de connexion 10.

De façon encore plus préférentielle, les branches d'ancrage 9 et le moyen d'immobilisation 11 sont venus de matière, formant ainsi un implant de fixation 7 monobloc. Un tel implant de fixation résiste particulièrement bien aux sollicitations mécaniques auxquelles est soumise l'articulation.

Avantageusement, et tel que cela est représenté sur les figures 3, 4, 9 et 10, les branches d'ancrage 9 sont pourvues de moyens anti-retour 16 spécifiquement conçus pour empêcher le déplacement de l'implant de fixation 7 suivant une direction S' opposée à son sens d'introduction S dans le greffon osseux 5. Avantageusement, les moyens anti-retour 16 sont préférentiellement formés par au moins une protubérance 22 faisant saillie sur la surface externe des branches d'ancrage 9. De façon encore plus préférentielle, les moyens anti-retour 16 sont formés par une pluralité de protubérances 22 disposées le long des branches d'ancrage 9, suivant la direction longitudinale X-X'.

Les branches d'ancrage 9 comportent avantageusement une face interne 9A, située sensiblement en regard du moyen d'immobilisation 11, sur laquelle sont disposés les moyens anti-retour 16. La face interne 9A présente ainsi un aspect cranté, chaque protubérance 22 formant un cran et présentant une surface inclinée 22A destinée à faciliter l'introduction des branches d'ancrage 9 dans les tissus osseux, et une face horizontale 22B, sensiblement perpendiculaire à la direction longitudinale X-X' et au sens S d'introduction de l'implant de fixation 7 de manière à empêcher le dégagement dudit implant de fixation 7 une fois ce dernier mis en place au sein de l'articulation 1.

Selon une caractéristique particulièrement avantageuse de l'invention, le moyen d'immobilisation 11 est formé par un organe de compression 30 adapté pour venir en appui contre le greffon osseux 5 et pour exercer sur ce dernier une pression suffisante pour que le greffon osseux 5 vienne, au moins partiellement, en appui contre les os 2, 3 de l'articulation 1 de manière à favoriser l'ostéosynthèse entre le greffon osseux 5 et les os 2, 3.

Dans le cas de la configuration illustrée sur la figure 5, où le greffon osseux est formé par un unique fragment d'os 5' de préférence monobloc, disposé au sein du logement 6, l'organe de compression 30 est adapté pour venir en appui contre au moins une partie de la surface externe 5'A du fragment d'os 5' de manière à comprimer ce dernier suivant un sens de compression F contre la paroi interne 6I du logement 6.

A cet effet, comme cela a été évoqué dans ce qui précède, l'organe de compression 30 est préférentiellement formé par une plaque 35, dont l'une des faces vient au contact de la surface externe 5'A du fragment d'os 5'. Le fragment d'os 5' peut avantageusement se présenter sous la forme d'un bloc hémi-cylindrique n'occupant pas la totalité du logement 6 présentant une portion de surface externe 5'A sensiblement plane contre laquelle la plaque 35 est susceptible de venir en appui.

Selon la méthode illustrée sur les figures 7 et 8, le greffon osseux 5 comporte au moins deux fragments d'os 2', 3' séparés par l'interstice I. L'organe de compression 30 est alors adapté pour être introduit au sein de l'interstice I, avec un faible jeu, et pour exercer une compression centrifuge ou radiale externe suivant les flèches F' sur les fragments d'os 2', 3', en vue de les repousser contre la paroi interne 6I du logement 6 et d'assurer ainsi l'expansion du greffon osseux 5 et son blocage au sein du logement 6, et plus généralement au sein de l'articulation 1.

Selon une caractéristique particulièrement avantageuse de l'invention, l'organe de compression 30 est pourvu de moyens d'écartement 32 progressif, adaptés pour assurer, au fur et à mesure de la pénétration de l'organe de compression 30 dans l'interstice I, la compression progressive des fragments d'os 2', 3'. Tel que cela est illustré sur la figure 10, les moyens d'écartement 32 sont avantageusement formés par une tranche 33 de l'organe de compression 30 dont l'épaisseur est variable. Ainsi, la tranche 33 s'étend, suivant le sens d'introduction S de l'organe de compression 30, entre une limite proximale 33A, située du coté de l'élément de connexion 10, et une limite distale 33B opposée. L'épaisseur de la tranche 33 augmente avantageusement, par exemple de façon continue, entre la limite distale 33B et la limite proximale 33A de manière à assurer l'écartement progressif des fragments d'os 2', 3'.

Il est cependant envisageable que l'épaisseur de la tranche 33 soit sensiblement constante, ou varie par paliers brusques, sans pour autant que l'on sorte du cadre de l'invention.

De façon particulièrement avantageuse, l'organe de compression 30 comporte une partie distale biseautée, destinée à faciliter son introduction dans le greffon osseux 5, et avantageusement formée par les moyens d'écartement 32. L'organe de compression 30 comprend en outre une partie proximale 34 sensiblement plus épaisse que sa partie distale qui correspond à la tranche 33. De façon encore plus préférentielle, la partie proximale 34 présente une épaisseur sensiblement égale à la largeur de l'interstice I, elle-même sensiblement identique à la largeur e de la fente articulaire 4 de manière à éviter un phénomène de raccourcissement du membre du paient comportant l'articulation 1.

Avantageusement, l'organe de compression 30 est formé par une plaque 35 sensiblement prismatique et aplatie formant un coin. La plaque 35 peut avantageusement être formée par un matériau à mémoire de forme, et être conçue pour s'expanser après son introduction au sein de l'interstice I en vue d'assurer la compression et l'écartement progressif des fragments d'os 2', 3'. La plaque 35 comporte de préférence deux faces 35A, 35B, au moins l'une desdites faces 35A comportant des rainures 36.

Selon une première variante de réalisation illustrée sur la figure 10, les rainures 36 peuvent avantageusement s'étendre suivant une direction sensiblement parallèle à la direction longitudinale X-X' d'extension de l'organe de compression 30. Une telle configuration permet notamment d'améliorer sensiblement l'efficacité de la compression.

Selon une autre variante illustrée sur la figure 9, les rainure 36 s'étendent de préférence suivant une direction sensiblement perpendiculaire à la direction longitudinale X-X' d'extension de l'organe de compression 30. Selon cette configuration, les rainures 36 forment avantageusement des moyens anti-retour s'opposant à l'extraction de l'implant une fois ce dernier introduit au sein du greffon osseux 5.

Selon une variante préférentielle illustrée notamment sur la figure 10, le moyen d'immobilisation 11 ou l'organe de compression 30, formé par la plaque 35, s'étend suivant un plan principal d'extension P et les branches d'ancrage 9 sont avantageusement situées dans ce même plan principal d'extension P.

Selon une caractéristique avantageuse de l'invention, l'implant de fixation 7 comporte des moyens de préhension 40 avantageusement formés par au moins une gorge et de préférence deux gorges 41 disposées de part et d'autre de l'implant de fixation 7, préférentiellement entre l'élément de connexion 10 et les moyens d'immobilisation 11.

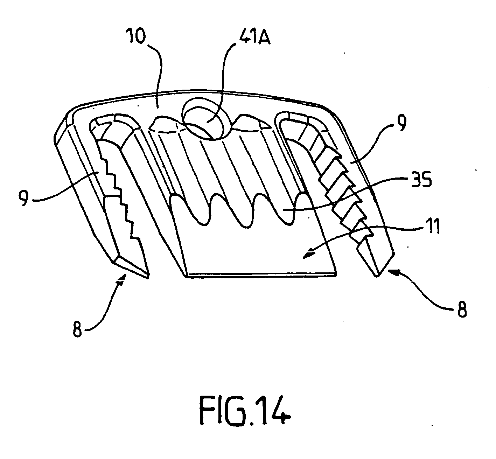

De façon alternative, tel que cela est illustré à la figure 14, les moyens de préhension 40 sont formés par un orifice 41A ménagé à travers l'épaisseur de l'implant, de préférence au voisinage de la jonction entre le moyen d'immobilisation et l'élément de connexion. Cet orifice 41A permet l'introduction d'un instrument d'extraction en forme de tige, permettant en particulier d'exercer un bras de levier sur l'implant pour l'extirper des os si nécessaire.

Dans un mode de réalisation particulièrement avantageux et qui constitue d'ailleurs une invention à part entière, l'organe de compression 30 est indépendant des éléments d'ancrage 8, c'est-à-dire qu'il n'est pas relié à ces derniers à l'aide de l'élément de connexion 10.

L'implant de fixation 7 est alors formé exclusivement par l'organe de compression 30, et ne comporte par l'élément d'ancrage 8.

Tel que cela est illustré sur les figures 11 à 13, l'organe de compression 30 comporte toutes les caractéristiques précédemment décrites mais est avantageusement destiné à être introduit au sein du greffon osseux 5 indépendamment des éléments d'ancrage 8. Il est alors possible d'utiliser d'autres moyens de fixation, par exemple des vis de fixation pour solidariser le fragment d'os 5' ou chacun des fragments 2', 3' aux os 2, 3, indépendamment de l'organe de compression 30.

L'organe de compression 30 est alors, selon le cas, spécifiquement adapté pour venir en appui contre les fragments d'os 5' ou 2', 3' et pour exercer une pression suffisante sur ces derniers pour les repousser contre la paroi interne 6I du logement 6 et assurer le blocage du greffon osseux 5 au sein de l'articulation 1.

Quel que soit leur mode de réalisation, l'implant de fixation 7, les éléments d'ancrage 8, le moyen d'immobilisation 11 ou l'organe de compression 30 sont avantageusement en matériau bio-résorbable, ce qui permet d'éviter une nouvelle intervention chirurgicale en vue de les retirer.

La méthode chirurgicale de mise en place de l'implant de fixation 7 va maintenant être décrite en se référant aux figures 1 à 13.

La méthode chirurgicale comprend, postérieurement à l'étape de mise en place ou de positionnement du greffon osseux 5 au sein de son logement 6, une étape de fixation du greffon osseux 5 à l'aide de l'implant de fixation 7 précédemment décrit.

Cette étape de fixation comporte tout d'abord une étape a) d'immobilisation relative des os 2, 3 formant l'articulation. Cette étape a) est réalisée à l'aide d'un ou plusieurs impacts, effectués par exemple à l'aide d'un impacteur approprié, sur l'implant, et par exemple soit sur l'élément de connexion 10, soit sur les éléments d'ancrage 8, de manière à faire pénétrer ces derniers dans chacun des os 2, 3 de l'articulation 1. Les os 2, 3 sont alors solidarisés l'un avec l'autre par l'intermédiaire de l'élément de connexion 10.

La méthode chirurgicale comprend en outre une étape b) de blocage du greffon osseux 5 vis-à-vis des os 2, 3 formant l'articulation. Cette étape b) peut être effectuée simultanément avec l'étape a) ou postérieurement à l'étape a), notamment lorsque le moyen d'immobilisation 11 n'est pas solidaire des éléments d'ancrage 8. Dans le cas où le moyen d'immobilisation 11 est formé par une pièce indépendante, il est possible d'introduire cette dernière au sein du greffon osseux 5 notamment à l'aide d'un ou plusieurs impacts effectués sur la partie proximale 11A du moyen d'immobilisation 11. Cette façon de procéder peut s'avérer par exemple judicieuse dans le cas où le moyen d'immobilisation 11 est formé par une plaque 35 prismatique formant un coin. En revanche, si le moyen d'immobilisation 11 est formé par une vis 15, l'étape b) sera préférentiellement réalisée en introduisant la vis 15 au sein du trou débouchant 19 puis en vissant cette dernière à l'intérieur du greffon osseux 5, par exemple au sein du fragment d'os 5' monobloc.

Dans le cas où le moyen d'immobilisation 11 est solidaire des éléments d'ancrage 8, le moyen d'immobilisation 11 est introduit au sein du greffon osseux 5 sensiblement en même temps que les éléments d'ancrage 8 pénètrent à l'intérieur des os 2, 3 sous l'action de l'impacteur.

La méthode chirurgicale comporte en outre avantageusement une étape c) de compression du greffon osseux 5 au sein du logement 6 ménagé de part et d'autre de la fente articulaire 4.

Avantageusement, les étapes a), b) et c) sont effectuées simultanément.

En particulier, le greffon osseux 5 étant formé par au moins deux fragments d'os 2', 3', l'étape c) implique d'exercer une compression centrifuge ou radiale externe suivant les flèches F' sur les fragments d'os 2', 3' en vue de les repousser vers la paroi interne 61 du logement 6, assurant ainsi leur blocage de même que celui du greffon osseux 5 d'une part au sein du logement 6 et d'autre part au sein de l'articulation 1.

Une telle étape peut être menée de manière indépendante des étapes a) et b) précédemment décrites et constitue alors une invention à part entière. Cette étape est avantageusement réalisée en introduisant l'organe de compression 30 au sein de l'interstice I entre les fragments d'os 2', 3', ledit organe de compression 30 étant ou non associé aux éléments d'ancrage 8 pour former l'implant de fixation 7.

L'implant de fixation 7 conforme à l'invention permet donc d'assurer, temporairement ou de façon permanente, un maintien efficace de l'articulation 1 et du greffon osseux 5 au sein de ladite articulation 1, et donc de faciliter l'ostéosynthèse entre le greffon osseux 5 et chacun des os 2, 3.

Un autre avantage de l'implant de fixation 7 conforme à l'invention provient de sa facilité de mise en place, à l'aide d'un simple impacteur, et sans qu'une incision supplémentaire ne soit nécessaire pour son implantation.

## Revendications

1. Implant de fixation d'un greffon osseux (5) disposé entre les os (2, 3) situés de part et d'autre d'une fente articulaire (4), en vue d'assurer l'arthrodèse d'une articulation (1), ledit implant de fixation (7) étant **caractérisé en ce qu'**il comporte :
- au moins deux éléments d'ancrage (8) destinés à être introduits dans les os (2, 3) formant ladite articulation (1), et pourvus d'une extrémité proximale (8A) et d'une extrémité distale (8B), ladite extrémité distale (8B) étant adaptée pour être introduite dans les os (2, 3), lesdits éléments d'ancrage (8) étant reliés entre eux par au moins un élément de connexion (10) s'étendant à l'extérieur de l'articulation (1),
- un moyen d'immobilisation (11) du greffon osseux (5), disposé entre les éléments d'ancrage (8) et relié à l'élément de connexion (10) de manière à assurer, en coopération avec les éléments d'ancrage (8), le blocage du greffon osseux (5) vis-à-vis des os (2, 3) de l'articulation (1) et vice-versa,
le moyen d'immobilisation (11) étant formé par une plaque (35) qui forme un organe de compression (30) comprenant une partie proximale (34) de la plaque sensiblement plus épaisse que la partie distale de la plaque , ledit organe de compression étant adapté pour exercer sur le greffon osseux (5) une pression radiale de manière à comprimer ce dernier contre les os (2, 3) de l'articulation (1).

2. Implant selon la revendication 1 **caractérisé en ce que** le moyen d'immobilisation (11) comporte un organe d'introduction (12) au sein du greffon osseux (5).

3. Implant selon la revendication 2 **caractérisé en ce que** le moyen d'immobilisation (11) s'étend, suivant une direction dite longitudinale (X-X'); entre une partie proximale (11A) reliée à l'élément de connexion (10), et une partie distale (11B) effilée, formant l'organe d'introduction.

4. Implant selon l'une des revendications 1 à 3 **caractérisé en ce que** le moyen d'immobilisation (11) comporte des moyens de blocage en rotation (13) adaptés pour empêcher la rotation du greffon osseux (5).

5. Implant selon la revendication 4 **caractérisé en ce que** les moyens de blocage en rotation (13) sont formés par au moins un méplat (14), ménagé le long du moyen d'immobilisation (11).

6. Implant selon l'une des revendications précédentes **caractérisé en ce que** le moyen d'immobilisation (11) est monté de façon amovible sur l'élément de connexion (10).

7. Implant selon l'une des revendications 1 à 5 **caractérisé en ce que** le moyen d'immobilisation (11) est solidarisé à demeure avec l'élément de connexion (10).

8. Implant selon l'une des revendications précédentes **caractérisé en ce que** le moyen d'immobilisation (11) est formé par un organe de compression (30) adapté pour venir en appui contre la surface externe (5'A) d'un fragment d'os (5'), formant le greffon osseux (5) et étant disposé au sein d'un logement (6) pourvu d'une paroi interne (6I) et ménagé dans les extrémités des os (2, 3) situés de part et d'autre de la fente articulaire (4), de manière à comprimer ledit fragment d'os (5') contre la paroi interne (6I) du logement (6).

9. Implant selon la revendication 8 **caractérisé en ce que** le moyen d'immobilisation (11) est formé par un organe de compression (30) adapté pour être introduit au sein d'un interstice (I) séparant le greffon osseux en au moins deux fragments d'os (2', 3'), et pour exercer une compression radiale externe sur les fragments d'os (2', 3') en vue de les repousser contre la paroi interne (6I) du logement (6) et assurer ainsi l'expansion du greffon osseux (5) et son blocage au sein de l'articulation (1).

10. Implant selon la revendication 9 **caractérisé en ce que** l'organe de compression (30) est pourvu de moyens d'écartement (32) progressif adaptés pour assurer, au fur et à mesure de sa pénétration dans l'interstice (I), la compression progressive des fragments d'os.

11. Implant selon la revendication 10 **caractérisé en ce que** les moyens d'écartement (32) sont formés par une tranche (33) de l'organe de compression, s'étendant, suivant le sens d'introduction (S) de l'organe de compression (30), entre une limite proximale (33A), située du côté de l'élément de connexion (10), et une limite distale (33B) opposée, l'épaisseur de ladite tranche (33) augmentant sensiblement entre la limite distale (33B) et la limite proximale (33A).

12. Implant selon l'une des revendications précédentes **caractérisé en ce que** l'organe de compression (30) comporte une partie distale biseautée, destinée à faciliter son introduction dans le greffon osseux (5).

13. Implant selon l'une des revendications précédentes **caractérisé en ce que** la partie proximale (34) de l'organe de compression (30) présente une épaisseur sensiblement égale à la largeur (e) de la fente articulaire (4).

14. Implant selon l'une des revendications précédentes **caractérisé en ce que** l'organe de compression (30) est formé par une plaque (35) sensiblement prismatique et aplatie.

15. Implant selon la revendication 14 **caractérisé en ce que** ladite plaque (35) comporte deux faces (35A, 35B) principales sensiblement parallèles, au moins l'une desdites faces (35A, 35B) comportant des rainures (36).

16. Implant selon la revendication 15 **caractérisé en ce que** les rainures (36) s'étendent suivant une direction sensiblement parallèle à la direction longitudinale (X-X') d'extension de l'organe de compression (30).

17. Implant selon la revendication 15 **caractérisé en ce que** les rainures (36) s'étendent suivant une direction sensiblement perpendiculaire à la direction longitudinale (X-X') d'extension de l'organe de compression (30).

18. Implant selon l'une des revendications précédentes **caractérisé en ce que** les éléments d'ancrage (8) sont formés par des branches d'ancrage (9), les extrémités distales (8B) desdites branches d'ancrage (9) étant sensiblement biseautées de manière à faciliter leur pénétration dans les tissus osseux.

19. Implant selon la revendication 18 **caractérisé en ce que** les branches d'ancrage (9) s'étendent longitudinalement de façon sensiblement parallèle à la direction longitudinale (X-X') d'extension du moyen d'immobilisation (11).

20. Implant selon la revendication 18 ou 19 **caractérisé en ce que** le moyen d'immobilisation (11) s'étend suivant un plan principal d'extension (P), et **en ce que** les branches d'ancrage (9) sont situées sensiblement dans ledit plan principal d'extension (P).

21. Implant selon l'une des revendications 18 à 20 **caractérisé en ce que** les branches d'ancrage (9) présentent, longitudinalement, une épaisseur variable.

22. Implant selon l'une des revendications 18 à 21 **caractérisé en ce que** les branches d'ancrage (9) ont sensiblement la même longueur que le moyen d'immobilisation (11).

23. Implant selon l'une des revendications 18 à 21 **caractérisé en ce que** les branches d'ancrage (9) ont des longueurs différentes.

24. Implant selon l'une des revendications 18 à 23 **caractérisé en ce que** les branches d'ancrage (9) sont pourvues de moyens anti-retour (16), spécifiquement conçus pour empêcher le déplacement de l'implant de fixation (7) suivant une direction (S') opposée à son sens d'introduction (S) dans le greffon osseux (5).

25. Implant selon la revendication 24 **caractérisé en ce que** les moyens anti-retour (16) sont formés par au moins une protubérance (22) faisant saillie sur la surface externe des branches d'ancrage (9).

26. Implant selon la revendication 25 **caractérisé en ce que** les moyens anti-retour (16) sont formés par une pluralité de protubérances (22), disposées le long des branches d'ancrage (9).

27. Implant selon l'une des revendications 24 à 26 **caractérisé en ce que** les branches d'ancrage (9) comportent une face interne (9A), située sensiblement en regard du moyen d'immobilisation (11), les moyens anti-retour (16) étant disposés sur ladite face interne (9A).

28. Implant selon l'une des revendications précédentes **caractérisé en ce qu'**il comporte des moyens de préhension (40).

29. Implant selon la revendication 28 **caractérisé en ce que** les moyens de préhension (40) sont formés par deux gorges (41), disposées de part et d'autre de l'implant de fixation (7).

30. Implant selon la revendication 28 **caractérisé en ce que** les moyens de préhension (40) sont formés par un orifice (41A) ménagé à travers l'épaisseur de l'implant, de préférence au voisinage de la Jonction entre le moyen d'immobilisation et l'élément de connexion.

31. Implant selon l'une des revendications précédentes **caractérisé en ce que** les moyens d'ancrage (8) sont formés par deux branches d'ancrage (9) latérales espacées, disposées en vis-à-vis de part et d'autre de élément de connexion (10), parallèlement l'une par rapport à l'autre, et **en ce que** le moyen d'immobilisation (11) est formé par une branche centrale sensiblement parallèle aux branches d'ancrage (9), lesdites branches d'ancrage (9) et ladite branche centrale s'étendant perpendiculairement par rapport à l'élément de connexion (10).

32. Implant selon l'une des revendications précédentes **caractérisé en ce que** l'élément de connexion (10) est agencé de manière à former un pont de connexion entre les éléments d'ancrage (8) chevauchant la fente articulaire (4).

## Claims

1. Fixation implant for a bone graft (5) arranged between bones (2, 3) located on both sides of an articular space (4) for the purpose of ensuring arthrodesis of a joint (1), said fixation implant (7) being **characterised in that** it comprises:
- at least two anchoring elements (8) designed to be introduced into the bones (2, 3) forming the joint, and equipped with a proximal end (8A) and a distal end (8B), said distal end (8B) being adapted to be introduced into the bones (2, 3), and said anchoring elements (8) being connected to each other by at least one connection element (10) extending outside the joint (1),
- an immobilisation means (11) for the bone graft (5), arranged between the anchoring elements (8) and connected to the connection element (10) in such a way as to ensure, in cooperation with the anchoring elements (8), blocking of the bone graft (5) with respect to the bones (2, 3) of the joint (1) and vice-versa,
the immobilisation means (11) being formed from a plate (35) forming a compression unit (30) comprising a proximal part (34) of the plate substantially thicker than the distal part of the plate, said compression unit being adapted to exert a radial pressure on the bone graft (5) so as to compress the latter against the bones (2, 3) of the joint (1).

2. Implant set forth in claim 1, **characterised in that** the immobilisation means (11) comprises a unit for introduction (12) within the bone graft (5).

3. Implant set forth in claim 2, **characterised in that** the immobilisation means (11) extend, in a direction referred to as longitudinal (X-X'), between a proximal part (11A) connected to the connection element (10), and a tapered distal part (11B), forming the introduction unit.

4. Implant set forth in one of claims 1 to 3, **characterized in that** the immobilisation means (11) comprises rotation blocking means (13) adapted to prevent rotation of the bone graft (5).

5. Implant set forth in claim 4, **characterised in that** the rotation blocking means (13) are formed from at least one flat part (14), arranged along the immobilisation means (11).

6. Implant set forth in one of the preceding claims, **characterised in** the immobilisation means (11) is mounted in a removable way on the connection element (10).

7. Implant set forth in one of claims 1 to 5, **characterised in that** the immobilisation means (11) is permanently united with the connection element (10).

8. Implant set forth in one of the preceding claims, **characterised in that** the immobilisation means (11) is formed from a compression unit (30), adapted to support the outer surface (5'13) of a bone fragment (5'), forming the bone graft (5) and arranged within a housing (6) being equipped with an inner wall (6I) and fitted in the ends of the bones (2, 3) located on both sides of the articular space (4), in such a way as to compress said bone fragment (5') against the inner wall (61) of the housing (6).

9. Implant set forth in claim 8, **characterised in that** the immobilisation means (11) is formed from a compression unit (30) adapted to be introduced within the interstice (I) which separates said bone graft into at least two bone fragments (2', 3'), and to exert an external radial compression on bone fragments (2', 3') for the purpose of pushing them back against the inner wall (61) of the housing (6) and thus ensuring the expansion of the bone graft (5) and its blocking within the joint (1).

10. Implant set forth in claim 9, **characterised in that** the compression unit (30) is equipped with progressive spreading means (32) adapted to ensure, as its penetration into the interstice (I) progresses, progressive compression of the bone fragments.

11. Implant set forth in claim 10, **characterised in that** the spreading means (32) are formed from a section (33) of the compression unit, extending, in the direction of introduction (S) of the compression unit (30), between a proximal limit (33A), located on the side of the connection element (10), and a distal limit (33B) opposite, the thickness of said section (33) increasing substantially between the distal limit (33B) and the proximal limit (33A).

12. Implant set forth in one of the preceding claims, **characterised in that** the compression unit (30) comprises a tapered distal part, designed to facilitate its introduction into the bone graft (5).

13. Implant set forth in one of the preceding claims, **characterised in that** the proximal part (34) of the compression unit (30) presents a thickness approximately equal to the width (e) of the articular space (4).

14. Implant set forth in one of the preceding claims, **characterised in that** the compression unit (30) is formed from an essentially prismatic and flattened plate (35).

15. Implant set forth in claim 14, **characterised in that** said plate (35) comprises two principal surfaces (35A, 35B) that are essentially parallel, at least one of said surfaces (35A, 35B) comprising grooves (36).

16. Implant set forth in claim 15, **characterised in that** the grooves (36) extend in a direction approximately parallel to the longitudinal extension direction (X-X') of the compression unit (30).

17. Implant set forth in claim 15, **characterised in that** the grooves (36) extend in a direction approximately perpendicular to the longitudinal extension direction (X-X') of the compression unit (30).

18. Implant set forth in one of the preceding claims, **characterised in that** the anchoring elements (8) are formed from anchoring arms (9), the distal ends (8B) of said anchoring arms (9) being substantially tapered so as to facilitate their penetration into the bone tissues.

19. Implant set forth in claim 18, **characterised in that** the anchoring arms (9) extend longitudinally essentially parallel to the longitudinal extension direction (X-X') of the immobilisation means (11).

20. Implant set forth in claim 18 or 19, **characterised in that** the immobilisation means (11) extends in a principal extension plane (P), and **in that** the anchoring arms (9) are located essentially in said principal extension plane (P).

21. Implant set forth in one of claims 18 to 20, **characterised in that** the anchoring arms (9) present, longitudinally, a variable thickness.

22. Implant set forth in one of claims 18 to 21, **characterised in that** the anchoring arms (9) are approximately the same length as the immobilisation means (11).

23. Implant set forth in one of claims 18 to 21, **characterised in that** the anchoring arms (9) have different lengths.

24. Implant set forth in one of claims 18 to 23, **characterised in that** the anchoring arms (9) are equipped with reverse-lock means (16), designed specifically to prevent displacement of the fixation implant (7) in a direction (S') opposite to its direction of introduction (S) into the bone graft (5).

25. Implant set forth in claim 24 **characterised in that** the reverse-lock means (16) are formed from at least one protuberance (22) projecting from the outer surface of the anchoring arms (9).

26. Implant set forth in claim 25, **characterised in that** the reverse-lock means (16) are formed from a plurality of protuberances (22) arranged along the anchoring arms (9).

27. Implant set forth in one of claims 24 to 26 **characterised in that** the anchoring arms (9) comprise an inner surface (9A), located approximately facing the immobilisation means (11), the reverse-lock means (16) being arranged on said inner surface (9A).

28. Implant set forth in one of the preceding claims, **characterised in that** it comprises gripping means (40).

29. Implant set forth in claim 28, **characterised in that** the gripping means (40) are formed from two grooves (41), arranged on both sides of the fixation implant (7).

30. Implant set forth in claim 28 **characterised in that** the gripping means (40) are formed from an orifice (41A) fitted through the thickness of the implant, preferably in the neighbourhood of the junction between the immobilisation means and the connection element.

31. Implant set forth in one of the preceding claims, **characterised in that** the anchoring means (8) are formed from two spaced-out lateral anchoring arms (9), arranged opposite each other on both sides of the connection element (10), parallel with respect to each other, and **in that** the immobilisation means (11) is formed from a central arm approximately parallel to the anchoring arms (9), said anchoring arms (9) and said central arm extending perpendicularly with respect to the connection element (10).

32. Implant set forth in one of the preceding claims, **characterised in that** the connection element (10) is arranged so as to form a connection bridge between the anchoring elements (8) which overlaps the articular space (4).

## Patentansprüche

1. Implantat zur Fixierung eines Knochentransplantats (5), das zwischen den Knochen (2, 3) angeordnet ist, die sich auf beiden Seiten einer Gelenkspalte (4) befinden, um die Arthrodese eines Gelenks (1) sicherzustellen, wobei das Fixierungsimplantat (7) **dadurch gekennzeichnet ist, dass** es Folgendes umfasst:
- mindestens zwei Verankerungselemente (8), die dazu gedacht sind, in die Knochen (2, 3), die das Gelenk (1) bilden, eingeführt zu werden, und die mit einem Proximalende (8A) und einem Distalende (8B) versehen sind, wobei das Distalende (8B) dazu geeignet ist, um in die Knochen (2, 3) eingeführt zu werden, wobei die Verankerungselemente (8) untereinander durch mindestens ein Verbindungselement (10) verbunden sind, das sich außerhalb des Gelenks (1) erstreckt,
- ein Mittel (11) zum Arretieren des Knochentransplantats (5), das zwischen den Verankerungselementen (8) angeordnet ist und mit dem Verbindungselement (10) derart verbunden ist, dass es mit den Verankerungselementen (8) zusammenwirkend die Blockierung des Knochentransplantats (5) gegenüber den Knochen (2, 3) des Gelenks (1) und umgekehrt sicherstellt,
wobei das Arretiermittel (11) durch ein Plättchen (35) gebildet wird, das ein Druckorgan (30) bildet, das einen Proximalteil (34) des Plättchens umfasst, der im Wesentlichen dicker ist als der Distalteil des Plättchens, wobei das Druckorgan dazu geeignet ist, um auf das Knochentransplantat (5) einen radialen Druck auszuüben, um dieses an den Knochen (2, 3) des Gelenks (1) zusammenzudrücken.

2. Implantat nach Anspruch 1, **dadurch gekennzeichnet, dass** das Arretiermittel (11) ein Einführungsorgan (12) im Innern des Knochentransplantats (5) umfasst.

3. Implantat nach Anspruch 2, **dadurch gekennzeichnet, dass** das Arretiermittel (11) sich in einer so genannten Längsrichtung (X-X') zwischen einem Proximalteil (11A), der mit dem Verbindungselement (10) verbunden ist, und einem spitz zu laufenden Distalteil (11 B), der das Einführungsorgan bildet, erstreckt.

4. Implantat nach einem der Ansprüche 1 bis 3, **dadurch gekennzeichnet, dass** das Arretiermittel (11) Mittel zum drehmäßigen Blockieren (13) umfasst, die dazu geeignet sind, die Drehung des Knochentransplantats (5) zu verhindern.

5. Implantat nach Anspruch 4, **dadurch gekennzeichnet, dass** die Drehblockierungsmittel (13) aus mindestens einer Abflachung (14), die an dem Arretiermittel (11) entlang eingerichtet ist, gebildet werden.

6. Implantat nach einem der vorhergehenden Ansprüche, **dadurch gekennzeichnet, dass** das Arretiermittel (11) abnehmbar auf dem Verbindungselement (10) montiert ist.

7. Implantat nach einem der Ansprüche 1 bis 5, **dadurch gekennzeichnet, dass** das Arretiermittel (11) mit dem Verbindungselement (10) permanent fest verbunden ist.

8. Implantat nach einem der vorhergehenden Ansprüche, **dadurch gekennzeichnet, dass** das Arretiermittel (11) durch ein Druckorgan (30) gebildet wird, das dazu geeignet ist, um sich an der Außenfläche (5'A) eines Knochenfragments (5') abzustützen, welches das Knochentransplantat (5) bildet und im Innern einer Aufnahme (6) angeordnet ist, die mit einer Innenwand (6I) versehen ist und in den Enden der Knochen (2, 3) eingerichtet ist, die sich auf beiden Seiten der Gelenkspalte (4) befinden, um das Knochenfragment (5') an der Innenwand (6I) der Aufnahme (6) zusammenzudrücken.

9. Implantat nach Anspruch 8, **dadurch gekennzeichnet, dass** das Arretiermittel (11) durch ein Druckorgan (30) gebildet wird, das dazu geeignet ist, um im Innern eines Zwischenraums (I) eingeführt zu werden, der das Knochentransplantat in mindestens zwei Knochenfragmente (2', 3') trennt, und um einen äußeren radialen Druck auf die Knochenfragmente (2', 3') auszuüben, um sie gegen die Innenwand (6I) der Aufnahme (6) zurückzudrängen und somit die Ausdehnung des Knochentransplantats (5) und seine Blockierung im Innern des Gelenks (1) sicherzustellen.

10. Implantat nach Anspruch 9, **dadurch gekennzeichnet, dass** das Druckorgan (30) mit Mitteln (32) zum allmählichen Spreizen versehen ist, die dazu geeignet sind, um das allmähliche Zusammendrücken der Knochenfragmente sicherzustellen, in dem Maße, wie es in den Zwischenraum (I) eindringt.

11. Implantat nach Anspruch 10, **dadurch gekennzeichnet, dass** die Spreizmittel (32) durch eine Scheibe (33) des Druckorgans gebildet werden, die sich entlang der Einführungsrichtung (S) des Druckorgans (30) zwischen einer Proximalgrenze (33A), die sich seitens des Verbindungselements (10) befinde, und einer gegenüberliegenden Distalgrenze (33B), erstreckt, wobei die Dicke der Scheibe (33) zwischen der Distalgrenze (33B) und der Proximalgrenze (33A) im Wesentlichen zunimmt.

12. Implantat nach einem der vorhergehenden Ansprüche, **dadurch gekennzeichnet, dass** das Druckorgan (30) einen abgeschrägten Distalteil trägt, der dazu geeignet ist, seine Einführung in das Knochentransplantat (5) zu erleichtern.

13. Implantat nach einem der vorhergehenden Ansprüche, **dadurch gekennzeichnet, dass** der Proximalteil (34) des Druckorgans (30) eine Dicke aufweist, die im Wesentlichen gleich der Breite (e) der Gelenkspalte (4) ist.

14. Implantat nach einem der vorhergehenden Ansprüche, **dadurch gekennzeichnet, dass** das Druckorgan (30) durch ein Plättchen (35) gebildet wird, das im Wesentlichen prismatisch und abgeflacht ist.

15. Implantat nach Anspruch 14, **dadurch gekennzeichnet, dass** das Plättchen (35) zwei im Wesentlichen parallele Hauptseiten (35A, 35B) umfasst, wobei mindestens eine der Seiten (35A, 353) Rillen umfasst (36).

16. Implantat nach Anspruch 15, **dadurch gekennzeichnet, dass** die Rillen (36) sich entlang einer Richtung erstrecken, die im Wesentlichen parallel zur Längserstreckungsrichtung (X X') des Druckorgans (30) ist.

17. Implantat nach Anspruch 15, **dadurch gekennzeichnet, dass** die Rillen (36) sich entlang einer Richtung erstrecken, die zu der Längserstreckungsrichtung (X-X') des Druckorgans (30) im Wesentlichen rechtwinklig ist.

18. Implantat nach einem der vorhergehenden Ansprüche, **dadurch gekennzeichnet, dass** die Verankerungselemente (8) durch Verankerungsschenkel (9) gebildet werden, wobei die Distalenden (8B) der Verankerungsschenkel (9) im wesentlichen abgeschrägt sind, um ihr Eindringen in das Knochengewebe zu erleichtern.

19. Implantat nach Anspruch 18, **dadurch gekennzeichnet, dass** die Verankerungsschenkel (9) sich der Länge nach im Wesentlichen parallel zur Längserstreckungsrichtung (X-X') des Arretiermittels (11) erstrecken.

20. Implantat nach Anspruch 18 oder 19, **dadurch gekennzeichnet, dass** das Arretiermittel (11) sich an einer Haupterstreckungsebene (P) entlang erstreckt, und dass die Verankerungsschenkel (9) sich im Wesentlichen in der Haupterstreckungsebene (P) befinden.

21. Implantat nach einem der Ansprüche 18 bis 20, **dadurch gekennzeichnet, dass** die Verankerungsschenkel (9) der Länge nach eine variable Dicke aufweisen.

22. Implantat nach einem der Ansprüche 18 bis 21, **dadurch gekennzeichnet, dass** die Verankerungsschenkel (9) im Wesentlichen die gleiche Länge wie das Arretiermittel (11) aufweisen.

23. Implantat nach einem der Ansprüche 18 bis 21, **dadurch gekennzeichnet, dass** die Verankerungsschenkel (9) unterschiedliche Längen aufweisen.

24. Implantat nach einem der Ansprüche 18 bis 23, **dadurch gekennzeichnet, dass** die Verankerungsschenkel (9) mit Rücklaufsperrmitteln (16) versehen sind, die spezifisch dazu ausgelegt sind, um die Verschiebung des Fixierungsimplantats (7) entlang einer Richtung (S'), die seiner Einführungsrichtung (S) in das Knochentransplantat (5) entgegengesetzt ist, zu verhindern.

25. Implantat nach Anspruch 24, **dadurch gekennzeichnet, dass** die Rücklaufsperrmittel (16) durch mindestens eine Ausstülpung (22) gebildet werden, die auf der Außenfläche der Verankerungsschenkel (9) hervorragt.

26. Implantat nach Anspruch 25, **dadurch gekennzeichnet, dass** die Rücklaufsperrmittel (16) durch eine Vielzahl von Ausstülpungen (22) gebildet werden, die an den Verankerungsschenkeln (9) entlang angeordnet sind.

27. Implantat nach einem der Ansprüche 24 bis 26, **dadurch gekennzeichnet, dass** die Verankerungsschenkel (9) eine Innenseite (9A) umfassen, die sich im Wesentlichen gegenüber dem Arretiermittel (11) befindet, wobei die Rücklaufsperrmittel (16) auf der Innenseite (9A) angeordnet sind.

28. Implantat nach einem der vorhergehenden Ansprüche, **dadurch gekennzeichnet, dass** es Greifmittel (40) umfasst.

29. Implantat nach Anspruch 28, **dadurch gekennzeichnet, dass** die Greifmittel (40) durch zwei Auskehlungen (41) gebildet werden, die sich auf beiden Seiten des Fixierungsimplantats (7) befinden.

30. Implantat nach Anspruch 28, **dadurch gekennzeichnet, dass** die Greifmittel (40) durch eine Öffnung (41A) gebildet werden, die durch die Dicke des Implantats hindurch eingerichtet ist, bevorzugt in der Nähe der Verbindungsstelle zwischen dem Arretiermittel und dem Verbindungselement.

31. Implantat nach einem der vorhergehenden Ansprüche, **dadurch gekennzeichnet, dass** die Verankerungsmittel (8) durch zwei beabstandete, seitliche Verankerungsschenkel (9) gebildet werden, die auf beiden Seiten des Verbindungselements (10) gegenüber und parallel zueinander angeordnet sind, und dass das Arretiermittel (11) aus einem mittleren Schenkel gebildet wird, der im Wesentlichen zu den Verankerungsschenkeln (9) parallel ist, wobei sich der Verankerungsschenkel (9) und der mittlere Schenkel rechtwinklig zum Verbindungselement (10) erstrecken.

32. Implantat nach einem der vorhergehenden Ansprüche, **dadurch gekennzeichnet, dass** das Verbindungselement (10) derart angeordnet ist, dass es eine Verbindungsbrücke zwischen den Verankerungselementen (8) bildet, welche die Gelenkspalte (4) überdeckt.
